# EUROPÄISCHE PATENTSCHRIFT

(11) **EP 1 432 787 B1**
(45) Veröffentlichungstag und Bekanntmachung des Hinweises auf die Patenterteilung: **14.09.2005**
(21) Anmeldenummer: 02800590.8
(22) Anmeldetag: 01.10.2002
(51) Int. Cl.: C12M 3/00, C12M 1/20, C12M 1/22, C12N 5/06

(54) **VORRICHTUNG UND VERFAHREN ZUR SELEKTION VON SELBSTBEWEGLICHEN BIOLOGISCHEN SPEZIES, INSBESONDERE VON SAMENZELLEN**
DEVICE AND METHOD FOR SELECTING LOCOMOTIVE BIOLOGICAL SPECIES, PARTICULARLY SPERM CELLS
DISPOSITIF ET PROCEDE POUR SELECTIONNER DES ESPECES BIOLOGIQUES A DEPLACEMENT AUTONOME, NOTAMMENT DES CELLULES DE SPERME

(30) Priorität: 05.10.2001 AT 15742001
(43) Veröffentlichungstag der Anmeldung: 30.06.2004
(73) Patentinhaber: ZECH, Josef, A-6020 Innsbruck (AT)
(72) Erfinder: ZECH, Josef, A-6020 Innsbruck (AT)
(74) Vertreter: Kador & Partner
(86) Internationale Anmeldenummer: PCT/EP2002/011019
(87) Internationale Veröffentlichungsnummer: WO 2003/031564

(56) Entgegenhaltungen:
- EP-A- 0 683 645
- FR-A- 2 539 628
- GB-A- 2 220 003
- ZECH J: "Homologous insemination - Heterologous insemination" JOURNAL FUR FERTILITAT UND REPRODUKTION 2002 AUSTRIA, Bd. 12, Nr. 2, 2002, Seiten 36-38, XP002254211 ISSN: 1019-066X
- MORTIMER D: "SPERM PREPARATION METHODS" JOURNAL OF ANDROLOGY, J.B. LIPPINCOTT CO., PHILADELPHIA, US, Bd. 21, Nr. 3, Mai 2000 (2000-05), Seiten 357-366, XP001018042 ISSN: 0196-3635

## Beschreibung

Die vorliegende Erfindung betrifft eine Vorrichtung zur Selektion von selbstbeweglichen biologischen Spezies, im besonderen zur Selektion von Samenzellen, die eine erste Kammer zur Aufnahme eines die zu selektierenden biologischen Spezies enthaltenden Mediums und, davon getrennt, eine zweite Kammer zur Aufnahme der selektierten Spezies in einem weiteren Medium umfasst.

Weiter betrifft die vorliegende Erfindung ein Verfahren zur Selektion von selbstbeweglichen biologischen Spezies, im besonderen von Samenzellen, sowie die Verwendung der erfindungsgemäßen Vorrichtung zur Selektion von selbstbeweglichen biologischen Spezies, im besonderen von Samenzellen.

Üblicherweise wird bei der extrakorpularen Befruchtung als ein erster Schritt die Gewinnung/Selektion von Samenzellen (Spermien) aus Samenflüssigkeit (Ejakulat) durchgeführt. Die selektierten Samenzellen werden dann in einem zweiten Schritt mit einer oder mehren Eizellen in-vitro oder in-vivo in Kontakt gebracht.

Bisher wurde die Selektion und Abtrennung von Samenzellen aus Samenflüssigkeit vorwiegend mittels Zentrifugation durchgeführt. Dieses Verfahren hat allerdings zum einen den Nachteil, dass die Samenzellen mechanisch stark belastet werden und es dadurch zur Beschädigung von (gesunden) Samenzellen kommt. Zum anderen können durch Zentrifugation bewegliche Samenzellen nur beschränkt selektiert und von wenig oder gar nicht beweglichen bzw. abgestorbenen getrennt werden.

Weiter ist zur Selektion von Samenzellen aus Samenflüssigkeit aus der EP 0 683 645 eine Vorrichtung bekannt, die zwei Kammern umfaßt, wobei die erste Kammer mit Samenflüssigkeit und die zweite Kammer mit einer Nährlösung zur Aufnahme der selektierten Samenzellen befüllt wird. Zwischen den Kammern wird eine Flüssigkeitsbrücke durch ein die Trennwand der Kammern übergreifendes Kapillarröhrchen oder durch loses Aufsetzen eines Brückenteils hergestellt. Die Selektion der Samenzellen erfolgt bei dieser Vorrichtung so, dass bewegliche Samenzellen über die Flüssigkeitsbrücke von der ersten zur zweiten Kammer gelangen können. Diese bekannte Vorrichtung hat allerdings die Nachteile, dass zum einen bei Verwendung eines Kapillarröhrchens die Selektion der Samenzellen nur sehr langsam vonstatten geht, während zum anderen bei Verwendung eines lose aufgesetzten Brückenteils die Flüssigkeitsbrücke nicht in vordefinierter, reproduzierbarer Weise gebildet wird, so dass häufig die Selektion der Samenzellen ebenfalls nur sehr langsam, wenn überhaupt, erfolgt.

Es ist eine Aufgabe der vorliegenden Erfindung, eine Vorrichtung zur Selektion von selbstbeweglichen biologischen Spezies, insbesondere von Samenzellen, zur Verfügung zu stellen, bei dem die selbstbeweglichen biologischen Spezies nicht mechanisch belastet werden und sich beispielsweise bewegliche Samenzellen von wenig oder gar nicht beweglichen Samenzellen abtrennen lassen.

Weiterhin ist es Aufgabe der vorliegenden Erfindung, eine solche Vorrichtung zur Verfügung zu stellen, bei der die Selektion der selbstbeweglichen biologischen Spezies möglichst rasch erfolgt und die eine einfache und sichere Handhabung durch Laborpersonal wie beispielsweise medizinischtechnische Assistenten gewährleistet.

Der vorliegenden Erfindung liegt die Erkenntnis zugrunde, dass eine solche Vorrichtung eine erste Kammer zur Aufnahme eines die zu selektierenden biologischen Spezies enthaltenden Mediums und eine zweite Kammer zur Aufnahme eines weiteren Mediums für die selektierten Spezies umfassen und zumindest einen flächenartigen Kanal aufweisen muss, der mit einem Medium, in dem sich die Spezies fortbewegen können, befüllbar ist und im befüllten Zustand der Vorrichtung die in der ersten und zweiten Kammer befindlichen Medien miteinander verbindet, wobei dieser Kanal unabhängig von der Handhabung der Vorrichtung durch den Benutzer in definierter Weise ausgebildet ist/wird.

Die vorliegende Erfindung stellt daher in einer ersten Ausführungsform eine Vorrichtung zur Verfügung, die zur Selektion von selbstbeweglichen biologischen Spezies, insbesondere von Samenzellen, eine erste Kammer zur Aufnahme eines die zu selektierenden Spezies enthaltenden Mediums und, davon getrennt, eine zweite Kammer zur Aufnahme der selektierten Spezies in einem weiteren Medium umfasst, und dadurch gekennzeichnet ist, dass sie ein auf die Kammern aufsetzbares Brückenelement umfasst, das wenigstens einen von Begrenzungswänden gebildeten, flächenartigen Kanal aufweist, der sich, wenn das Brückenelement aufgesetzt ist, von der ersten Kammer zur zweiten Kammer erstreckt, jeweils im Bereich der ersten Kammer und der zweiten Kammer eine Öffnung aufweist und mit einem Medium, in dem sich die selbstbeweglichen biologischen Spezies fortbewegen können, so befüllbar ist, dass das Medium in der ersten Kammer mit dem in der zweiten Kammer verbunden ist.

Im Sinne der vorliegenden Erfindung werden unter selbstbeweglichen biologischen Spezies solche organischen Spezies wie beispielsweise Mikroorganismen oder, insbesondere, Samenzellen (Spermien) verstanden, die sich selbstständig in einem Medium, wie beispielsweise einer Flüssigkeit, fortbewegen können.

Unter dem Begriff flächenartiger Kanal wird im Rahmen der vorliegenden Erfindung ein Hohlraum verstanden, der in einer Dimension eine geringere Ausdehnung als in den anderen zwei Dimensionen aufweist. Vorzugsweise beträgt die Ausdehnung des Hohlraumes in den anderen zwei Dimensionen ein Vielfaches der Ausdehnung in der ersten Dimension.

Unter dem Begriff Medium wird im Sinne der Erfindung jede Art von Materie verstanden, in der sich die zu selektierenden Spezies fortbewegen können. Bevorzugte Medien sind fluide Medien wie beispielsweise Flüssigkeiten sowie Gele. In diesen fluiden Medien und Gelen können auch weitere Substanzen wie beispielsweise Nährstoffe für die biologischen Spezies und Elektrolyte vorhanden sein.

Beispielsweise kann bei der Selektion von Samenzellen das die zu selektierenden Spezies enthaltende Medium Samenflüssigkeit und das die selektierten Samenzellen aufnehmende Medium eine Nährlösung sein. In diesem Fall ist zur Befüllung des Kanals ebenfalls Nährlösung das bevorzugte Medium.

Die Handhabung der Vorrichtung beispielsweise zur Selektion von Samenzellen aus Samenflüssigkeit erfolgt so, dass vom Benutzer zunächst in eine erste Kammer die Samenflüssigkeit eingefüllt wird. Im Anschluss daran wird das Brückenelement auf die Kammern aufgesetzt und die zweite Kammer sowie der Kanal/die Kanäle mit Nährlösung befüllt, wobei vorzugsweise auch die Sammenflüssigkeit in der ersten Kammer mit Nährlösung überschichtet wird. Im befüllten Zustand entsteht so eine Flüssigkeitsbrücke zwischen der ersten und der zweiten Kammer, in der keine Konvektion der darin enthaltenen Flüssigkeit auftritt. Über diese Brücke können dann bewegliche Samenzellen aus der Samenflüssigkeit in die Nährlösung der zweiten Kammer wandern. Damit sind die Samenzellen bei der Selektion keiner mechanischen Belastung ausgesetzt und es werden die beweglichen von den unbeweglichen Samenzellen abgetrennt. Die in der zweiten Kammer enthaltenen selektierten Samenzellen können dann beispielsweise mit einer Pipette für die extrakorporale Befruchtung zur Insemination, Kryokonservation oder für Diagnosezwecke entnommen werden. Es ist auch möglich, eine oder mehrere Eizellen zur Befruchtung direkt in die zweite Kammer einzubringen.

In der erfindungsgemäßen Vorrichtung dieser ersten Ausführungsform weist das aufsetzbare Brückenelement selbst wenigstens einen flächenartigen Kanal auf, der bereits in definierter Form ausgebildet ist, d.h. dass er feststehende Abmessungen besitzt, die durch die fixierte Anordnung der Begrenzungswände vordefiniert sind. Dies bedeutet, dass sich im befüllten Zustand die Verbindung der Kammern durch das im Kanal vorhandene Medium nach Aufsetzen des Brückenelements auf die Kammern in optimaler Weise ausbilden kann. Dies erfolgt unabhängig von der Handhabung der Vorrichtung durch das Laborpersonal. Damit ist gewährleistet, dass die Selektion der selbstbeweglichen biologischen Spezies, insbesondere der Samenzellen, immer in reproduzierbarer, rascher Weise erfolgt.

Ein weiterer Vorteil der erfindungsgemäßen Vorrichtung ist, dass das Medium in den Kanälen der Vorrichtung reicht, d.h. keiner Konvektion unterworfen ist, so dass die zu selektierenden Spezies nur aufgrund ihrer ???beweglichkeit selektiert werden und sich somit beispielsweise die für eine Befruchtung aktivsten Samenzellen gewinnen lassen.

Bei der mit Hilfe der erfindungsgemäßen Vorrichtung durchgeführten Selektion von Samenzellen aus Samenflüssigkeit läßt sich bereits nach etwa 15 bis 30 Minuten in die zweite Kammer der Vorrichtung eine genügend große Menge an beweglichen Samenzellen selektieren, die dann beispielsweise für die extrakorporale Befruchtung ausreichen.

In einer zweiten Ausführungsform der vorliegenden Erfindung umfasst die Vorrichtung zur Selektion von selbstbeweglichen biologischen Spezies, insbesondere von Samenzellen, eine erste Kammer zur Aufnahme eines die zu selektierenden Spezies enthaltenden Mediums und, davon getrennt, eine zweite Kammer zur Aufnahme der selektierten Spezies in einem weiteren Medium, wobei die Wände der ersten Kammer und der zweiten Kammer zumindest teilweise miteinander verbunden sind und ein Brückenelement so auf die Kammern aufsetzbar ist, dass die verbundenen Wände und eine Seite des Brückenelements je eine Begrenzungswand eines flächenartigen Kanals bilden, der jeweils im Bereich der ersten Kammer und der zweiten Kammer eine Öffnung aufweist und mit einem Medium, in dem sich die selbstbeweglichen biologischen Spezies fortbewegen können, so befüllbar ist, dass das Medium in der ersten Kammer mit dem in der zweiten Kammer verbunden ist und dadurch gekennzeichnet ist, dass wenigstens ein Justierelement so vorgesehen ist, dass der Kanal beim Aufsetzen des Brückenelements immer in gleicher, definierter Weise gebildet wird.

Bei dieser zweiten Ausführungsform der erfindungsgemäßen Vorrichtung wird ein flächenartiger Kanal beim Aufsetzen des Brückenelements auf die Verbindungswand der Kammern durch den Benutzer zuverlässig immer in vordefinierter, reproduzierbarer Weise gebildet, d.h. der Kanal besitzt unabhängig von der Handhabung durch den Benutzer die durch die Anordnung der Begrenzungswände in Verbindung mit dem Justierelement vordefinierten Abmessungen. Durch das Justierelement ist es möglich, dem gebildeten Kanal eine für die Ausbildung der Flüssigkeitsbrücke optimale Form zu geben. Damit lassen sich auch mit dieser Ausführungsform der vorliegenden Erfindung die bereits für die erste Ausführungsform angegebenen Vorteile erreichen und somit eine rasche und reproduzierbare Selektion von selbstbeweglichen biologischen Spezies, unabhängig von der Handhabung der Vorrichtung durch den Benutzer, erzielen.

Vorzugsweise ist die erfindungsgemäße Vorrichtung der ersten Ausführungsform so ausgebildet, dass die Wände der ersten Kammer und der zweiten Kammer zumindest teilweise miteinander verbunden sind und das Brückenelement so auf die Kammern aufsetzbar ist, dass die verbundenen Wände und eine Seite des Brückenelements je eine Begrenzungswand eines flächenartigen Kanals bilden, der jeweils im Bereich der ersten Kammer und der zweiten Kammer eine Öffnung aufweist und mit einem Medium, in dem sich die selbstbeweglichen biologischen Spezies fortbewegen können, so befüllbar ist, dass das Medium in der ersten Kammer mit dem in der zweiten Kammer verbunden ist.

Durch diese bevorzugte Ausführungsform sind im aufgesetzten Zustand des Brückenelements zumindest zwei flächenartige Kanäle zwischen der ersten und der zweiten Kammer vorhanden, in denen sich eine Verbindung von erster und zweiter Kammer durch ein Medium ausbilden kann.

Weiter bevorzugt ist in der Vorrichtung dieser Ausführungsform ein Justierelement so vorgesehen, dass der durch die verbundenen Wände der Kammern und einer Seite des Brückenelements als Begrenzungswände gebildete Kanal beim Aufsetzen des Brückenelements immer in gleicher, definierter Weise gebildet wird.

Durch diese bevorzugte Ausführungsform der erfindungsgemäßen Vorrichtung, die die Merkmale der oben beschriebenen ersten und zweiten Ausführungsform miteinander verbindet, wird gewährleistet, dass auch der zweite Kanal, der durch das Aufsetzen des Brückenelements auf die Kammern durch den Benutzer gebildet wird, immer in vordefinierter, reproduzierbarer Weise gebildet wird und somit die Selektion der beweglichen biologischen Spezies noch effektiver und rascher erfolgen kann.

Die nachfolgend beschriebenen bevorzugten Ausführungsformen beziehen sich auf alle vorstehend beschriebenen Ausführungsformen der erfindungsgemäßen Vorrichtung.

In einer bevorzugten Ausführungsform der erfindungsgemäßen Vorrichtung weist das auf die Kammern aufsetzbare Brückenelement wenigstens zwei, weiter bevorzugt wenigstens drei und noch mehr bevorzugt wenigstens vier von Begrenzungswänden gebildete, flächenartige Kanäle auf, die sich, wenn das Brückenelement aufgesetzt ist, von der ersten Kammer zur zweiten Kammer erstrecken, jeweils im Bereich der ersten Kammer und der zweiten Kammer eine Öffnung aufweisen und mit einem Medium, in dem sich die selbstbeweglichen biologischen Spezies fortbewegen können, so befüllbar ist, dass das Medium in der ersten Kammer mit dem in der zweiten Kammer gebildet wird.

Durch diese bevorzugten Ausführungsformen der erfindungsgemäßen Vorrichtung wird zum einen gewährleistet, dass die Selektion der selbstbeweglichen biologischen Spezies noch effizienter und rascher vonstatten geht, und zum anderen, dass bei Ausfall eines Kanals beispielsweise durch Verschmutzung oder durch unvollständige Bildung der Verbrückung der beiden Kammern durch das im Kanal vorhandene Medium, etwa durch Luftbläschen in einem Flüssigkeitsmedium, trotzdem eine Selektion erfolgen kann.

In einer weiteren bevorzugten Ausführungsform der erfindungsgemäßen Vorrichtung ist der Abstand der einen Kanal bildenden, einander gegenüberliegenden Begrenzungswände oder Teilen davon so vordefiniert, dass auf das Medium, mit dem der Kanal befüllt wird, Kapillarkräfte wirken, so dass sich der Kanal beim Kontakt mit dem Medium zumindest teilweise, bevorzugterweise vollständig, selbständig vollsaugt.

Dieser die Kapillarkräfte bewirkende Abstand entspricht der oben erwähnten ersten Dimension der flächenartigen Kanäle. Durch diese Ausführungsform wird das Befüllen des Kanals mit Medium erleichtert.

Weiter bevorzugt ist die erfindungsgemäße Vorrichtung gemäß einer der vorstehenden Ausführungsformen so ausgebildet, dass die einen Kanal bildenden, einander gegenüberliegenden Begrenzungswände oder Teile von Begrenzungswänden einen maximalen Abstand von 1,0 mm, vorzugsweise von 0,5 mm, aufweisen.

Weiter bevorzugt weisen die einen Kanal bildenden, einander gegenüberliegenden Begrenzungswände oder Teile von Begrenzungs-wänden einen minimalen Abstand von 0,1 mm, vorzugsweise von 0,2 mm auf.

Besonders bevorzugt weisen einander gegenüberliegenden Begrenzungswände einen Abstand im Bereich von 0,3 bis 0,4 mm auf.

Die angegebenen bevorzugten Abstände der Begrenzungswände entsprechen der oben erwähnten ersten Dimension der Kanäle. Durch die angegebenen bevorzugten Werte ist eine optimale Ausbildung vorzugsweise einer Flüssigkeitsbrücke eines wäßrigen Mediums oder einer Nährlösung in den Kanälen durch die Kapillarwirkung der Kanäle sichergestellt.

Weiter bevorzugt verlaufen bei der erfindungsgemäßen Vorrichtung die einander gegenüberliegenden Begrenzungswände jeweils eines Kanals zueinander parallel.

In einer weiteren bevorzugten Ausführungsform sind die Kanäle der erfindungsgemäßen Vorrichtung so ausgebildet, dass jeweils zwei Kanäle zumindest teilweise eine gemeinsame Begrenzungswand aufweisen. Weiter bevorzugt haben jeweils zwei Kanäle der Vorrichtung eine gesamte Begrenzungswand gemein.

Dies ermöglicht eine besonders einfache und materialsparende Ausführung der erfindungsgemäßen Vorrichtung.

Vorzugsweise weisen die Begrenzungswände der Kanäle und damit auch die Kanäle der Vorrichtung selbst einen U-förmigen Querschnitt auf.

Weiter bevorzugt ragen die Kanäle der erfindungsgemäßen Vorrichtung weiter in die zweite Kammer als in die erste Kammer hinein. Dadurch wird die Handhabung der Vorrichtung vereinfacht.

In einer weiteren bevorzugten Ausführungsform ragt zumindest je ein Kanal der Vorrichtung im Vergleich zu den anderen Kanälen unterschiedlich weit in die erste Kammer hinein. Weiter bevorzugt ragen alle Kanäle, die die Vorrichtung aufweist, unterschiedlich weit in die erste Kammer hinein.

Durch die verschiedenen Eintauchtiefen der Kanäle in die erste Kammer wird erreicht, dass der Austritt speziell eines Mediums mit höherer Oberflächenspannung durch eine verbesserte Tropfenbildung aus den Kanälen erleichtert wird und damit die Befüllung der Kanäle erleichtert wird. Dies trifft beispielsweise auf Nährflüssigkeit für Samenzellen zu, die im Vergleich zu Wasser eine erheblich höhere Oberflächenspannung aufweist.

Weiter bevorzugt ist die erfindungsgemäße Vorrichtung so beschaffen, dass eine der Kammern ringförmig ausgebildet ist und die andere, zweite Kammer umschließt.

Vorzugsweise sind die Kammern und das Brückenelement der erfindungsgemäßen Vorrichtung rotationssymmetrisch ausgebildet.

Weiter bevorzugt sind die beiden Kammern der Vorrichtung Teil eines aus einem Stück gefertigten Kammerelements.

Die erfindungsgemäße Vorrichtung kann aus Materialien wie beispielsweise Glas oder Kunststoff aufgebaut sein. Vorzugsweise wird jedoch Kunststoff, weiter bevorzugt transparenter Kunststoff, verwendet.

Die Verwendung von Kunststoff als Material für die erfindungsgemäße Vorrichtung weist den Vorteil auf, dass die Vorrichtung in einfacher Weise und damit ökonomisch günstig herstellbar ist. Aufgrund dieses ökonomischen Vorteils ist es für den Anwender möglich, die erfindungsgemäße Vorrichtung nach einmaligem Gebrauch zu entsorgen, womit eine hygienisch bedenkliche Wiederverwendung entfällt. Zum anderen kann die erfindungsgemäße Vorrichtung in einfacher Weise, beispielsweise durch Elektronenbeschuss, sterilisiert werden.

Die erfindungsgemäße Vorrichtung kann beispielsweise für den Transport oder die Aufbewahrung in einen äußeren Behälter eingebracht werden, der vorzugsweise einen Bodenteil und einen Deckel umfaßt.

Weiter bevorzugt ist der Behälter so ausgebildet, dass die erfindungsgemäße Vorrichtung im zusammengesetzten Zustand darin ortsfixiert ist.

Die vorliegende Erfindung stellt weiterhin ein Verfahren zur Selektion von selbstbeweglichen biologischen Spezies, insbesondere von Samenzellen, zur Verfügung, worin die zu selektierende Spezies in einem Medium in einer ersten Kammer einer Vorrichtung vorgelegt werden und die selektierten Spezies in einer zweiten Kammer der Vorrichtung in einem weiteren Medium aufgenommen werden, dadurch gekennzeichnet, dass die Medien in den Kammern der Vorrichtung über ein in einem von Begrenzungswänden gebildeten flächenartigen Kanal der in vordefinierter Weise gebildet wird, vorhandenes Medium verbunden werden, wobei die selbstbeweglichen Spezies durch ihre Eigenbewegung über das in dem Kanal vorhandene Medium von der ersten in die zweite Kammer gelangen können und somit selektiert werden.

In bevorzugten Ausführungsformen des erfindungsgemäßen Verfahrens wird eine Vorrichtung gemäß einer der vorstehend beschriebenen Ausführungsformen verwendet.

Weiterhin betrifft die vorliegenden Erfindung auch die Verwendung einer Vorrichtung gemäß einer der vorstehend beschriebenen Ausführungsformen zur Selektion von in einer Flüssigkeit enthaltenen selbstbeweglichen biologischen Spezies, insbesondere zur Selektion von in Samenflüssigkeit enthaltenen Samenzellen.

Im folgenden wird ein Ausführungsbeispiels der erfindungsgemäßen Vorrichtung anhand der beigefügten Figuren beschrieben. Es zeigen:

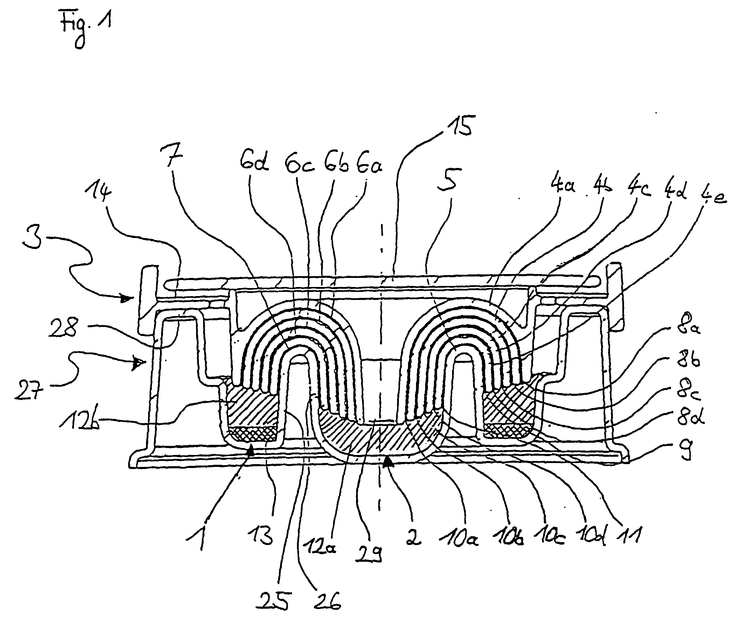

- Fig. 1:: einen Querschnitt der erfindungsgemäßen Vorrichtung im zusammengesetzten Zustand, und

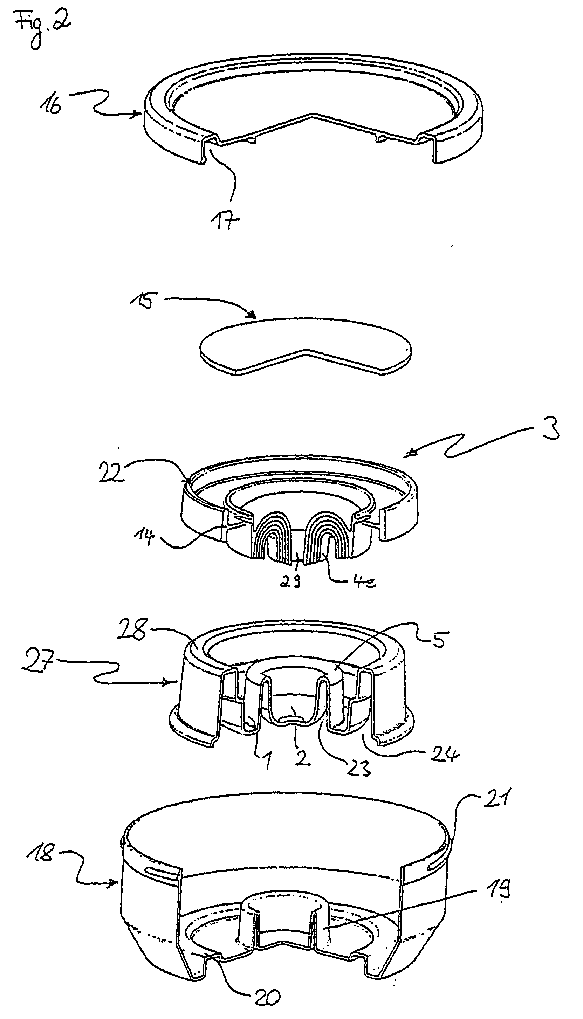

- Fig. 2:: teilgeschnittene Schrägansichten der erfindungsgemäßen Vorrichtung sowie eines Behälters dafür.

Die erfindungsgemäße Vorrichtung dieses Ausführungsbeispiels wird gebildet durch ein Brückenelement (3), das vier definiert vorgebildete Kanäle (6a-d) umfasst, sowie durch ein Kammerelement (27), das eine ringförmige erste Kammer (1) zur Aufnahme beispielsweise von Samenflüssigkeit (13) und eine von dieser ringförmigen Kammer vollständig umschlossene zweite Kammer (2) zur Aufnahme von Nährflüssigkeit (12a) aufweist.

Das Brückenelement (3) weist insgesamt fünf Begrenzungswände (4a-e) auf, die einen U-förmigen Querschnitt besitzen und die ringförmige Verbindung (5) der inneren Außenwand der ringförmigen ersten Kammer (25) und der äußeren Wand der zweiten, zentralen Kammer (26) ringförmig übergreifen. Die Begrenzungswände (4a-e) sind dabei sämtlich zueinander parallel angeordnet und weisen untereinander einen Abstand von etwa 0,3 mm auf. Die parallele Ausrichtung der Begrenzungsflächen (4a-e) und die genaue Beabstandung können beispielsweise durch punktförmige Verbindungen zwischen den Begrenzungswänden (4a-e) erreicht werden.

Durch die Begrenzungswände (4a-e) werden die vier Kanäle (6a-d) gebildet, die sich sämtlich Teil des Brückenelements sind. Ein weiterer Kanal (7) wird durch die innere Fläche der Begrenzungswand (4e) des Brückenelements (3) und der äußeren Oberfläche der verbundenen Wände der Kammern (5) gebildet, wenn das Brückenelement (3) auf das Kammerelement (27) aufgesetzt ist.

Durch das am Brückenelement (3) vorhandene Justierelement (14), das als T-förmiger Ring ausgeführt ist und formschlüssig auf die obere Umfangswand (28) des Kammerelements (27) aufgesetzt werden kann, ist gewährleistet, dass das Brückenelement (3) vom Benutzer immer in genau definierter Weise, zentriert auf das Kammerelement (27) aufsetzbar ist, wodurch der gleichbleibende Abstand der geraden Teile der Begrenzungswände (4e) und (5) des Kanals (7) gewährleistet ist. Weiterhin wird durch das Justierelement (14) gewährleistet, dass das Brückenelement (3) auch immer in definierter, vorbestimmter Höhe auf das Kammerelement (27) aufgesetzt wird, so dass sich auch im Umlenkbereich der Begrenzungswände (4e) und (5) ebenfalls der vorbestimmte Abstand zwischen den Begrenzungswänden einstellt. Durch die ringförmige Ausbildung des Justierelements (14) wird auch erreicht, dass die erste, ringförmige Kammer (1) im zusammengesetzten Zustand der Vorrichtung vollständig bedeckt ist. Damit läßt sich eine Verschmutzung der Samenflüssigkeit vermeiden.

Weiterhin ist ein Deckel (15) vorhanden, der nach Zusammensetzen und Befüllen der Vorrichtung auf das Brückenelement (3) aufgesetzt werden kann. Dadurch werden Verschmutzungen der in der zweiten Kammer (2) vorhandenen Nährlösung vermieden.

Zur Durchführung der Selektion von Samenzellen aus Samenflüssigkeit wird in die ringförmige Kammer (1) Samenflüssigkeit mit zu selektierenden Samenzellen (13) so eingefüllt, dass die Samenflüssigkeit im zusammengesetzten Zustand der Vorrichtung noch nicht an die Öffnungen (8a-d, 9) der Kanäle auf Seiten der ringförmigen Kammer (1) heranreicht. Im Anschluß daran wird das Brückenelement (3) auf die Kammern (1, 2) aufgesetzt.

In die zentrale Kammer (2) wird dann durch die zentrale Öffnung (29) Nährflüssigkeit (12a) so eingefüllt, dass sich die Kanäle (6a-d, 7) aufgrund des höheren Flüssigkeitsniveaus in der zweiten Kammer (2) bzw. des beim Befüllen auf die Flüssigkeit ausgeübten Drucks, beispielsweise mit einer Pipette, und der in den Kanälen wirkenden Kapillarkräfte vollständig mit Nährflüssigkeit vollsaugen und die Samenflüssigkeit (13) der ringförmigen Kammer (1) von Nährflüssigkeit (12b) überschichtet wird.

Sobald das Überschichten der Samenflüssigkeit (13) mit Nährflüssigkeit (12b) beendet ist, ruht die Nährflüssigkeit in den Kanälen, d.h. ist keiner Konvenktion unterworfen, und es beginnen die beweglichen Samenzellen aufgrund ihrer Eigenbewegung durch die als Flüssigkeitsbrücken fungierenden gefüllten Kanäle (6a-d, 7) in die Nährlösung (12a) der zentralen Kammer (2) zu wandern.

Die Begrenzungswände (4a-e) des Brückenelements (3) sind so ausgebildet, dass die Öffnung (8a) des Kanals (6a) die geringste Eintauchtiefe in die ringförmige erste Kammer (1) besitzt und die Eintauchtiefe der Öffnungen (8b,c,d, 9) der Kanäle (6b,c,d, 7) in die erste Kammer (1) stetig zunimmt.

Umgekehrt nimmt die Eintauchtiefe der Öffnungen (10a-d, 11) der Kanäle (6a-d, 7) in die zweite, zentrale Kammer (2) stetig ab.

Zum Schutz der erfindungsgemäßen Vorrichtung vor Kontaminationen, beispielsweise beim Transport, ist ein Behälter (18) mit einem passenden Deckel (16) vorgesehen, wobei dieser Behälter zur Ortsfixierung der Vorrichtung ringförmige Stege (19) bzw. (2) aufweist, die in entsprechende Aussparungen (23) bzw. (24) des Kammerelements (27) eingreifen können. Der Deckel (16) weist eine ringförmige Aussparung (17) auf, die formschlüssig auf den profilierten ringförmigen Rand (22) des mit dem Brückenelement (3) verbundenen Zentrierelement (14) aufsetzbar ist. Der Deckel kann beispielsweise über einen Schraubverschluss (21) verschlossen werden.

## Patentansprüche

1. Vorrichtung zur Selektion von selbstbeweglichen biologischen Spezies, insbesondere von Samenzellen, die eine erste Kammer (1) zur Aufnahme eines die zu selektierenden Spezies enthaltenden Mediums (12b, 13) und, davon getrennt, eine zweite Kammer (2) zur Aufnahme der selektierten Spezies in einem weiteren Medium (12a) umfasst, **dadurch gekennzeichnet,**
**dass** sie ein auf die Kammern (1,2) aufsetzbares Brückenelement (3) umfasst, das wenigstens einen von Begrenzungswänden (4a-e) gebildeten, flächenartigen Kanal (6a-d) aufweist, der sich, wenn das Brückenelement (3) aufgesetzt ist, von der ersten Kammer (1) zur zweiten Kammer (2) erstreckt, jeweils im Bereich der ersten Kammer (8a-d) und der zweiten Kammer (10a-d) eine Öffnung aufweist und mit einem Medium, in dem sich die selbstbeweglichen biologischen Spezies fortbewegen können, so befüllbar ist, dass das Medium in der ersten Kammer (12b, 13) mit dem in der zweiten Kammer (12a) verbunden ist.

2. Vorrichtung zur Selektion von selbstbeweglichen biologischen Spezies, insbesondere von Samenzellen, die eine erste Kammer (1) zur Aufnahme eines die zu selektierenden Spezies enthaltenden Mediums (12b, 13) und, davon getrennt, eine zweite Kammer (2) zur Aufnahme der selektierten Spezies in einem weiteren Medium (12a) umfasst, wobei die Wände der ersten Kammer (1) und der zweiten Kammer (2) zumindest teilweise miteinander verbunden sind (5) und ein Brückenelement (3) so auf die Kammern (1,2) aufsetzbar ist, dass die verbundenen Wände (5) und eine Seite des Brückenelements (4e) je eine Begrenzungswand eines flächenartigen Kanals (7) bilden, der jeweils im Bereich der ersten Kammer (9) und der zweiten Kammer (11) eine Öffnung aufweist und mit einem Medium, in dem sich die selbstbeweglichen biologischen Spezies fortbewegen können, so befüllbar ist, dass das Medium in der ersten Kammer (12b, 13) mit dem in der zweiten Kammer (12a) verbunden ist, **dadurch gekennzeichnet,**
**dass** wenigstens ein Justierelement (14) so vorgesehen ist, dass der Kanal (7) beim Aufsetzen des Brückenelements (3) immer in gleicher, definierter Weise gebildet wird.

3. Vorrichtung nach Anspruch 1, **dadurch gekennzeichnet,**
**dass** die Wände der ersten Kammer (1) und der zweiten Kammer (2) zumindest teilweise miteinander verbunden sind (5) und das Brückenelement (3) so auf die Kammern (1,2) aufsetzbar ist, dass die verbundenen Wände (5) und eine Seite des Brückenelements (4e) je eine Begrenzungswand eines flächenartigen Kanals (7) bilden, der jeweils im Bereich der ersten Kammer (9) und der zweiten Kammer (11) eine Öffnung aufweist und mit einem Medium, in dem sich die selbstbeweglichen biologischen Spezies fortbewegen können, so befüllbar ist, dass das Medium in der ersten Kammer (12b, 13) mit dem in der zweiten Kammer (12a) verbunden ist.

4. Vorrichtung nach Anspruch 3, **dadurch gekennzeichnet,**
**dass** wenigstens ein Justierelement (14) so vorgesehen ist, dass der durch die verbundenen Wände der Kammern (5) und einer Seite des Brückenelements (4e) als Begrenzungswände gebildete Kanal (7) beim Aufsetzen des Brückenelements (3) immer in gleicher, definierter Weise gebildet wird.

5. Vorrichtung nach einem der vorstehenden Ansprüche, **dadurch gekennzeichnet,**
**dass** der Abstand der einen Kanal bildenden, einander gegenüberliegenden Begrenzungswände (4a-e, 5) oder Teile der Begrenzungswände (4a-e, 5) so vordefiniert ist, dass auf das Medium, mit dem der Kanal befüllt wird, Kapillarkräfte wirken, so dass sich der Kanal bei Kontakt mit dem Medium selbstständig ganz oder teilweise vollsaugt.

6. Vorrichtung nach einem der vorstehenden Ansprüche, **dadurch gekennzeichnet,**
**dass** die einen Kanal (6a-d,7) bildenden, einander gegenüberliegenden Begrenzungswände (4a-e, 5) oder Teile von Begrenzungswänden (4a-e, 5) einen maximalen Abstand von 1,0 mm, vorzugsweise von 0,5 mm und einen minimalen Abstand von 0,1 mm, vorzugsweise von 0,2 mm aufweisen.

7. Vorrichtung nach einem der vorstehenden Ansprüche, **dadurch gekennzeichnet,**
**dass** die einander gegenüberliegenden Begrenzungswände (4a-e,5) jeweils eines Kanals (6a-d,7) zueinander parallel verlaufen.

8. Vorrichtung nach einem der vorstehenden Ansprüche, **dadurch gekennzeichnet,**
**dass** jeweils zwei Kanäle (6a-d,7) zumindest teilweise eine gemeinsame Begrenzungswand (4b-e) aufweisen.

9. Vorrichtung nach einem der vorstehenden Ansprüche, **dadurch gekennzeichnet,**
**dass** die Begrenzungswände (4a-e,5) der Kanäle (6a-d,7) U-förmigen Querschnitt aufweisen.

10. Vorrichtung nach einem der vorstehenden Ansprüche, **dadurch gekennzeichnet,**
**dass** eine der Kammern (1,2) ringförmig ausgebildet ist (1) und die andere Kammer (2) umschließt.

11. Verfahren zur Selektion von selbstbeweglichen biologischen Spezies, insbesondere von Samenzellen, worin die zu selektierenden Spezies in einem Medium in einer ersten Kammer (1) einer Vorrichtung vorgelegt werden und die selektierten Spezies in einer zweiten Kammer (2) der Vorrichtung in einem weiteren Medium aufgenommen werden, **dadurch gekennzeichnet,**
**dass** die Medien in den Kammern der Vorrichtung über ein in einem von Begrenzungswänden (4a-e, 5) gebildeten flächenartigen Kanal (6a-d, 7), der mit vordefinierten Abmessungen gebildet wird, vorhandenem Medium verbunden werden, wobei die selbstbeweglichen Spezies durch ihre Eigenbewegung über das in dem Kanal vorhandene Medium von der ersten in die zweite Kammer gelangen können und somit selektiert werden.

12. Verfahren nach Anspruch 11, **dadurch gekennzeichnet,**
**dass** eine Vorichtung nach einem der Ansprüche 1 bis 10 verwendet wird.

13. Verwendung einer Vorrichtung nach einem der Ansprüche 1 bis 10 zur Selektion von selbstbeweglichen biologischen Spezies, insbesondere zur Selektion von Samenzellen.

## Claims

1. Device for the selection of motile biological species, in particular of spermatogenic cells, which comprises a first chamber (1) for receiving a medium (12b, 13) containing the species to be selected and, separated from it, a second chamber (2) for receiving the selected species in a further medium (12a), **characterized in that**
it comprises a bridge element (3) which can be fitted onto the chambers (1, 2) which has at least one flat duct (6a-d) formed by boundary walls (4a-e) which, when the bridge element (3) is fitted, extends from the first chamber (1) to the second chamber (2), has in each case an opening in the region of the first chamber (8a-d) and the second chamber (10a-d) and can be filled with a medium in which the motile biological species can move such that the medium in the first chamber (12b, 13) is connected to that in the second chamber (12a).

2. Device for the selection of motile biological species, in particular of spermatogenic cells, which comprises a first chamber (1) for receiving a medium (12b, 13) containing the species to be selected and, separated from it, a second chamber (2) for receiving the selected species in a further medium (12a), the walls of the first chamber (1) and the second chamber (2) being at least partially connected to each other (5) and a bridge element being able to be fitted onto the chambers (1, 2) such that the connected walls (5) and one side of the bridge element (4e) each form a boundary wall of a flat duct (7) which has an opening in each case in the region of the first chamber (9) and the second chamber (11) and can be filled with a medium in which the motile biological species can move such that the medium in the first chamber (12b, 13) is connected to that in the second chamber (12a), **characterized in that** at least one adjusting element (14) is provided such that the duct (7) is always formed in the same, defined, manner when the bridge element (3) is fitted.

3. Device according to claim 1, **characterized in that**
the walls of the first chamber (1) and the second chamber (2) are at least partially connected (5) to one another and the bridge element (3) can be fitted onto the chambers (1, 2) such that the connected walls (5) and one side of the bridge element (4e) each form a boundary wall of a flat duct (7) which has an opening in each case in the region of the first chamber (9) and the second chamber (11) and can be filled with a medium in which the motile biological species can move such that the medium in the first chamber (12b, 13) is connected to that in the second chamber (12a).

4. Device according to claim 3, **characterized in that**
at least one adjusting element (14) is provided such that the duct (7) formed by the connected walls of the chambers (5) and one side of the bridge element (4e) as boundary walls is always formed in the same, defined, manner when the bridge element (3) is fitted.

5. Device according to one of the previous claims, **characterized in that**
the distance between the opposite boundary walls (4a-e, 5) or parts of the boundary walls (4a-e, 5) forming a duct is predefined such that capillary forces act on the medium with which the duct is filled, with the result that the duct automatically fully or partially soaks itself upon contact with the medium.

6. Device according to one of the previous claims, **characterized in that**
the maximum distance between the opposite boundary walls (4a-e, 5) or parts of boundary walls (4a-e, 5) forming a duct (6a-d, 7) is at most 1.0 mm, preferably 0.5 mm, and the minimum distance at least 0.1 mm, preferably 0.2 mm.

7. Device according to one of the previous claims, **characterized in that**
the opposite boundary walls (4a-e, 5) of a duct (6a-d, 7) in each case run parallel to each other.

8. Device according to one of the previous claims, **characterized in that**
two ducts (6a-d, 7) each have, at least partially, a common boundary wall (4b-e).

9. Device according to one of the previous claims, **characterized in that**
the boundary walls (4a-e, 5) of the ducts (6a-d, 7) have a U-shaped cross-section.

10. Device according to one of the previous claims, **characterized in that**
one of the chambers (1, 2) is developed annular (1) and encloses the other chamber (2).

11. Method for the selection of motile biological species, in particular spermatogenic cells, in which the species to be selected are introduced into a medium in a first chamber (1) of a device, and the selected species are housed in a further medium in a second chamber (2) of the device,
**characterized in that**
the media in the chambers of the device are connected via a medium present in a flat duct (6a-d, 7) formed by boundary walls (4a-e, 5), which is formed with predefined dimensions, the motile species being able to travel as a result of their own movement via the medium present in the chamber from the first into the second chamber, and thus be selected.

12. Method according to claim 11, **characterized in that**
a device according to one of claims 1 to 10 is used.

13. Use of a device according to one of claims 1 to 10 for the selection of motile biological species, in particular for the selection of spermatogenic cells.

## Revendications

1. Dispositif en vue de la sélection d'espèces biologiques capables de se mouvoir de manière autonome, en particulier de spermatozoïdes, qui comprend une première chambre (1) en vue de la réception d'un milieu contenant les espèces à sélectionner (12b, 13) et, séparée de cette dernière, une deuxième chambre (2) en vue de la réception des espèces sélectionnées dans un milieu supplémentaire (12a), **caractérisé**
**en ce qu'**il comprend un élément faisant pont (3) pouvant être placé sur les chambres (1,2), qui présente au moins un canal de forme plate (6a-d), formé par des parois de limitation (4a-e), qui, une fois l'élément faisant pont (3) mis en place, se prolonge de la première chambre (1) à la deuxième chambre (2), qui présente, à chaque fois, dans la zone de la première chambre (8a-d) et de la deuxième chambre (10a-d), un orifice, et qui peut être rempli avec un milieu, dans lequel les espèces biologiques capables de se mouvoir de manière autonome peuvent se déplacer, de telle sorte que le milieu dans la première chambre (12b, 13) est relié à celui dans la deuxième chambre (12a).

2. Dispositif en vue de la sélection d'espèces biologiques capables de se mouvoir de manière autonome, en particulier de spermatozoïdes, qui comprend une première chambre (1) en vue de la réception d'un milieu contenant les espèces à sélectionner (12b, 13) et, séparée de cette dernière, une deuxième chambre (2) en vue de la réception des espèces sélectionnées dans un milieu supplémentaire (12a), les parois de la première chambre (1) et de la deuxième chambre (2) étant au moins partiellement reliées les unes aux autres (5) et un élément faisant pont (3) pouvant être placé sur les chambres (1,2) de telle sorte que les parois reliées (5) et un côté de l'élément faisant pont (4e), forment respectivement une paroi de limitation d'un canal de forme plate (7), qui présente, à chaque fois, dans la zone de la première chambre (9) et de la deuxième chambre (11), un orifice, et qui peut être rempli d'un milieu, dans lequel les espèces biologiques capables de se mouvoir de manière autonome peuvent se déplacer, de sorte que le milieu dans la première chambre (12b, 13) est relié à celui de la deuxième chambre (12a), **caractérisé**
**en ce que** l'on prévoit au moins un élément de réglage (14), de manière à ce que, lors de la mise en place de l'élément faisant pont (3), le canal (7) se forme toujours d'une façon définie identique.

3. Dispositif selon la revendication 1, **caractérisé**
**en ce que** les parois de la première chambre (1) et de la deuxième chambre (2) sont au moins partiellement reliées les unes aux autres (5) et en ce que l'élément faisant pont (3) peut être placé sur les chambres (1,2) de telle sorte que les parois reliées (5) et un côté de l'élément faisant pont (4e) forment, respectivement, une paroi de limitation d'un canal (7) de forme plate, qui présente, à chaque fois, dans la zone de la première chambre (9) et de la deuxième chambre (11), un orifice, et qui peut être rempli d'un milieu, dans lequel les espèces biologiques, capables de se mouvoir de manière autonome peuvent se déplacer, de telle sorte que le milieu dans la première chambre (12b, 13) est relié avec celui dans la deuxième chambre(12a).

4. Dispositif selon la revendication 3, **caractérisé**
**en ce que** l'on prévoit au moins un élément de réglage (14), de manière à ce que, lors de la mise en place de l'élément faisant pont (3), le canal (7) formé en tant que parois de limitation par les parois reliées des chambres (5) et par un côté de l'élément faisant pont (4e), se forme toujours d'une façon définie identique.

5. Dispositif selon l'une quelconque des revendications précédentes, **caractérisé**
**en que** l'écart entre les parois de limitation (4a-e, 5), se faisant face l'une l'autre, formant un canal ou entre des parties des parois de limitation (4a-e, 5) est défini au préalable de telle sorte que des forces capillaires agissent sur le milieu avec lequel le canal est rempli, de manière à ce que le canal lorsqu'il entre en contact avec le milieu se remplisse, d'une manière autonome, par aspiration complètement ou partiellement.

6. Dispositif selon l'une quelconque des revendications précédentes, **caractérisé**
**en ce que** les parois de limitation (4a-e, 5), se faisant face l'une l'autre, formant un canal (6a-d,7) ou des parties des parois de limitation (4a-e, 5) présentent un écart maximal de 1,0 mm, de préférence, de 0,5 mm et un écart minimal de 0,1 mm, de préférence, de 0,2 mm.

7. Dispositif selon l'une quelconque des revendications précédentes, **caractérisé**
**en ce que** les parois de limitation (4a-e,5), se faisant face l'une l'autre, de chaque canal respectif (6a-d,7), s'étendent d'une manière parallèle les unes aux autres.

8. Dispositif selon l'une quelconque des revendications précédentes, **caractérisé**
**en ce qu'**à chaque fois deux canaux (6a-d,7) présentent au moins partiellement une paroi de limitation commune (4b-e).

9. Dispositif selon l'une quelconque des revendications précédentes, **caractérisé**
**en ce que** les parois de limitation (4a-e,5) des canaux (6a-d,7) présentent une section transversale en forme de U.

10. Dispositif selon l'une quelconque des revendications précédentes, **caractérisé**
**en ce que** l'une des chambres (1,2) est formée d'une manière annulaire (1) et en ce qu'elle entoure l'autre chambre (2).

11. Procédé en vue de la sélection d'espèces biologiques capables de se mouvoir de manière autonome, en particulier de spermatozoïdes, dans lequel les espèces à sélectionner sont placées au préalable dans un milieu dans une première chambre (1) d'un dispositif et en ce que les espèces sélectionnées sont reçues dans un milieu supplémentaire dans une deuxième chambre (2) du dispositif, **caractérisé,**
**en ce que** les milieux dans les chambres du dispositif sont reliés par l'intermédiaire d'un milieu présent dans un canal de forme plate (6a-d, 7), formé par les parois de limitation (4a-e, 5), lequel canal est réalisé avec des dimensions prédéfinies, les espèces capables de se mouvoir de manière autonome pouvant parvenir de la première à la deuxième chambre par leurs propres mouvements par l'intermédiaire du milieu présent dans le canal et pouvant ainsi être sélectionnées.

12. Procédé selon la revendication 11, **caractérisé**
**en ce que** l'on utilise un dispositif selon l'une des revendications 1 à 10.

13. Utilisation d'un dispositif selon l'une quelconque des revendications 1 à 10, en vue de la sélection d'espèces biologiques capables de se mouvoir de manière autonome, en particulier en vue de la sélection de spermatozoïdes.
